# EUROPÄISCHE PATENTSCHRIFT

(11) **EP 0 897 990 B1**
(45) Veröffentlichungstag und Bekanntmachung des Hinweises auf die Patenterteilung: **07.10.2009**
(21) Anmeldenummer: 98115491.7
(22) Anmeldetag: 18.08.1998
(51) Int. Cl.: C12Q 1/68

(54) **Reduktion von Kreuzkontaminationen bei Nukleinsäureamplifikationen**
Reduction of cross contamination within nucleic acid amplifications
Réduction de contamination croisée concernant l'amplification des acides nucléiques

(30) Priorität: 20.08.1997 DE 19736062
(43) Veröffentlichungstag der Anmeldung: 24.02.1999
(73) Patentinhaber: Roche Diagnostics GmbH, 68305 Mannheim (DE)
(72) Erfinder: Haberhausen, Gerd, Dr., 82377 Penzberg (DE); Jäger, Stephan, Dr., 82377 Penzberg (DE); Sobek, Harald, Dr., 82377 Penzberg (DE)
(74) Vertreter: Herren, Barbara

(56) Entgegenhaltungen:
- EP-A- 0 522 884
- WO-A-99/25867
- US-A- 5 418 149
- US-A- 5 466 591
- US-A- 5 536 649
- US-A- 5 624 833
- THORNTON C G ET AL: "Utilizing uracil DNA glycosylase to control carryover contamination in PCR: Characterizationof residual UDG activity following thermal cycling" BIOTECHNIQUES, EATON PUBLISHING, NATICK, US, Bd. 13, Nr. 2, 1992, Seite 180,182,184 XP002113111 ISSN: 0736-6205

### SEQUENZPROTOKOLL

(1) ALLGEMEINE ANGABEN:
   (i) ANMELDER:
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEFON: 621 759 4348
      (H) TELEFAX: 621 759 4457
   (ii) BEZEICHNUNG DER ERFINDUNG: Reduktion von Kreuzkontaminationen bei Nukleinsäureamplifikationen
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 26 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (xi) SEQUENZBESCHREIBUNG: SEQ ID NO: 1:
      CCTCTTCCCC AGAACAATAA GAACAC 26
(2) ANGABEN ZU SEQ ID NO: 2:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (ix) MERKMAL:
      (A) NAME/SCHLÜSSEL: misc_feature
      (B) LAGE: 1
      (D) SONSTIGE ANGABEN:/note= "Biotinmarkiert mit Biotin-Aminolinker"
   (xi) SEQUENZBESCHREIBUNG: SEQ ID NO: 2:
      GGGATTCCTG TAACAACAAG TCAGG 25
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (ix) MERKMAL:
      (A) NAME/SCHLÜSSEL: misc_feature
      (B) LAGE: 1
      (D) SONSTIGE ANGABEN:/note= "Am 5'-Phosphat Rutheniumtribispyridylmarkiert"
   (xi) SEQUENZBESCHREIBUNG: SEQ ID NO: 3:
      CATAGCACTA TAGAACTCTG 20
(2) ANGABEN ZU SEQ ID NO: 4:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (xi) SEQUENZBESCHREIBUNG: SEQ ID NO: 4:
      AGTTGGAGGA CATCAAGCAG CCATGCAAAT 30
(2) ANGABEN ZU SEQ ID NO: 5:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (ix) MERKMAL:
      (A) NAME/SCHLÜSSEL: misc_feature
      (B) LAGE:1
      (D) SONSTIGE ANGABEN:/note= "Mit Aminolinker am 5'-Phosphat biotinmarkiert"
   (xi) SEQUENZBESCHREIBUNG: SEQ ID NO: 5:
      TGCTATGTCA GTTCCCCTTG GTTCTCT 27
(2) ANGABEN ZU SEQ ID NO: 6:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodeoxyribonukleotid"
   (ix) MERKMAL:
      (A) NAME/SCHLÜSSEL: misc_feature
      (B) LAGE: 1
      (D) SONSTIGE ANGABEN:/note= "am 5'-Phosphat Rutheniumkomplexmarkiert"
   (xi) SEQUENZBESCHREIBUNG: SEQ ID NO: 6:
      ATCAATGAGG AAGCTGCAGA 20

## Beschreibung

Gegenstand der Erfindung sind Verfahren zur Amplifikation von Nukleinsäuren, Verfahren zum Nachweis von Nukleinsäuren mit Hilfe dieser Amplifikationsverfahren, die Verwendung von Lauroylsarkosin zur Verhinderung der Reaktivierung von Uracil-N-Glycosilase sowie ein Reagenz und ein Reagenzkit zur Durchführung dieses Verfahrens.

Seit der Einführung von Verfahren zur Amplifikation von Nukleinsäuren (z. B. der Polymerase-Kettenreaktion (PCR), wie beschrieben in US-A-4,683,202) in die Nukleinsäurediagnostik sind eine ganze Reihe von Analyten zum ersten Mal analytisch erfaßbar geworden. Diese Verfahren sind in der Lage, selbst in geringsten Konzentrationen vorhandene Analytnukleinsäuren, z. B. von HCV, soweit zu vermehren, daß sie solchen Nukleinsäuretests zugänglich sind, die bislang hochkonzentrierten Analyten vorbehalten waren. Allerdings hat es sich mit der Zeit herausgestellt, daß die Labors, in denen die Amplifikationen durchgeführt wurden, mittlerweile schon so stark mit den amplifizierten Nukleinsäuren verseucht sind, daß Tests in Proben, die eigentlich die niedrigkonzentrierte Nukleinsäure gar nicht enthalten, zu falschpositiven Ergebnissen führen, da die Proben durch die Umgebung mit Nukleinsäuren aus vorhergehenden Amplifikationen verseucht wurden (Kreuzkontaminationen). Durch die hohe Sensitivität der auf Amplifikation beruhenden Nukleinsäuretests können selbst geringste Verseuchungen nachgewiesen und somit vorgetäuscht werden, daß der Analyt in der Probe enthalten ist (falschpositive Ergebnisse).

In EP-B-0 566 050 ist ein Verfahren zur Stabilisierung von alkalischen nukleinsäurehaltigen Lösungen mit anionischen, nicht-ionischen und zwitterionischen Detergenzien beschrieben.

In EP-A-0 401 037 ist ein Verfahren beschrieben, das dem beschriebenen Mangel teilweise abhilft. In diesem Verfahren werden während der Amplifikation in die Amplifikate jeder Analytnukleinsäure nicht natürlicherweise in der nachzuweisenden Nukleinsäure enthaltene Mononukleotide eingebaut. Vor Durchführung einer folgenden Amplifkation wird die Probe, zusammen mit den verwendeten Reagenzien, einer Vorbehandlung unterzogen, in der alle eingeschleppten Amplifikate früherer Amplifikationen enzymatisch abgebaut werden. Beispielhaft sind als Abbaureagenz Uracil-N-Glycosilase (UNG) und als modifizierender Baustein für die Amplifikate dUTP genannt. Ein alternatives Verfahren benutzt anstelle von uracilhaltigen Mononukleotiden uracilhaltige Primer. Ein solches Verfahren, in dem die Primerbindungsstellen auf früher erzeugten Amplikaten abgebaut werden, ist in EP-A-0 415 755 beschrieben.

Der Mechanismus dieser Dekontaminationsverfahren beruht auf dem spezifischen Erkennen Uracil-haltiger Amplifikate, die durch das Enzym abgebaut werden. Beim Ansetzen der Amplifikationsreaktion wird UNG der Probe meist schon mit dem Mastermix, welcher alle für die Amplifikation erforderlichen Reagenzien enthält, zugegeben. Vor der anschließenden Amplifikation findet in einem kurzen Inkubationsschritt die oben genannte Abbaureaktion statt. Erwärmt man anschließend das Reaktionsgemisch auf eine Temperatur von über ca. 40°C, so wird die UNG inaktiviert. Dies ist notwendig, um zu gewährleisten, daß die UNG nicht die neu synthetisierte DNA abbaut, die im Verlaufe der Amplifikation akkumuliert.

Erfolgt im Anschluß an die Amplifikation jedoch nicht direkt die Nachweisreaktion, so kommt es zu einer Rückfaltung des denaturierten Enzyms und damit verbunden zu einer Wiedergewinnung seiner Aktivität (BioTechniques 13, 181-183, 1992). Hierdurch kann es zu falschnegativen Testergebnissen kommen. Zur Vermeidung der UNG-Reaktivierung werden bisher unterschiedliche Verfahren angewendet. In einer ersten Variante werden die Amplifikate bei erhöhten Temperaturen (z. B. >50°C) aufbewahrt. Hierdurch wird jedoch entweder ein zusätzliches temperierbares Gerät benötigt oder der Thermocyclerplatz wird durch das Reaktionsgefäß bis zur Weiterführung des Tests blockiert. Andererseits kommt es unter diesen Bedingungen mit der Zeit auch zu einem temperaturbedingten Zerfall des Amplifikats . In einer weiteren Variante werden die Amplifikate bei niedrigen Temperaturen (z. B. 4°C) aufbewahrt. Auch diese Variante führt zu zusätzlichen Hardwareanforderungen und damit zu erhöhten Kosten. In einer dritten Variante wird der Reaktionslösung nach Durchführung der Amplifikation eine Stoplösung (zumeist NaOH) zugesetzt. Das Zupipettieren einer Stoplösung bedeutet jedoch eine verminderte Convenience für den Bearbeiter und ist grundsätzlich, ob manuell oder automatisch ausgeführt, mit einem Öffnen des Reaktionstubes verbunden. Mit jedem zusätzlichen Arbeitsschritt, insbesondere nach Amplifikation, steigt jedoch auch die Kontaminationsgefahr beträchtlich.

Ein weiterer Ansatz ist in US 5,418,149 beschrieben, wobei Methoden zur Amplifikation von Nukleinsäuren offenbart werden, bei denen nichtspezifische Amplifikation und Kontaminationseffekte auf Grund von Produkten früheren Amplifikationen reduziert werden.

Aufgabe der vorliegenden Erfindung war es daher, die Nachteile des Standes der Technik ganz oder teilweise zu vermeiden, und insbesondere ein einfacheres Verfahren zur Amplifikation mit reduziertem Kontaminationsrisiko bereitzustellen.

Gegenstand der Erfindung ist ein Verfahren zur Amplifikation von Nukleinsäuren in einer Probe enthaltend die Schritte
- Behandlung der Probe mit einem Enzym unter Bedingungen, bei denen Produkte aus Amplifikationen von Nukleinsäuren anderer Proben enzymatisch abgebaut werden,
- Inaktivierung des Enzyms, und
- Behandlung der Probe unter Bedingungen, bei denen die Nukleinsäuren amplifiziert werden,
wobei die Amplifikation in Anwesenheit eines die Reaktivierung des Enzyms verhindernden Reagenzes stattfindet.

Ebenfalls Gegenstand der Erfindung ist ein Reagenz zum Einsatz in obigem Verfahren und die Verwendung von Detergenzien in Verfahren zur Amplifikation von Nukleinsäuren.

In FIG 1 ist schematisch ein beispielhaftes Nachweisverfahren gezeigt, bei dem das Reagenz der Probe schon vor Amplifikation zugesetzt wird.

In FIG 2 ist schematisch ein Verfahren gezeigt, bei dem das Reagenz nach Amplifikation zugesetzt wird.

Unter einem Detergenz im Sinne der vorliegenden Erfindung wird eine grenzflächenaktive Substanz verstanden, die prinzipiell anionisch, kationisch, ampholytisch oder nicht-ionisch aufgebaut sein kann. Bevorzugt sind anionische Detergenzien.

Kern der vorliegenden Erfindung ist die Erkenntnis, daß es möglich ist, die Reaktivierung von Abbauenzymen nach einer Amplifikation durch Zugabe eines Reagenz schon vor der Amplifikation zu unterdrücken. Ein Fachmann hätte nicht erwartet, daß es ein Reagenz geben könnte, das zwar die Reaktivierung des Enzyms verhindert, aber die Aktivität des nativen Enzyms nicht wesentlich negativ beeinflußt.

Das erfindungsgemäße Amplifikationsverfahren verwendet eine Probe, in der die zu amplifizierende Nukleinsäure enthalten ist. Meist wird das erfindungsgemäße Verfahren in der Analytik eingesetzt werden, weshalb die in der Probe enthaltene Nukleinsäure auch Analytnukleinsäure genannt werden wird. Es sind jedoch auch andere Möglichkeiten der Verwendung für die erfindungsgemäß amplifizierten Nukleinsäuren gegeben, z. B. die anschließende rekombinante Klonierung oder die Sequenzierung. Unter Analytnukleinsäuren sind Nukleinsäuren jeglichen Ursprungs zu verstehen, beispielsweise Nukleinsäuren viroiden, viralen, bakteriellen oder zellulären Ursprungs. Sie können in Lösung, Suspension, aber auch an Festkörpern fixiert oder in zellhaltigen Medien, Zellabstrichen, fixierten Zellen, Gewebsschnitten oder fixierten Organismen vorliegen. Bevorzugt liegen die Nukleinsäuren in Lösung vor.

Sofern die Nukleinsäure noch nicht zugänglich ist, wird sie vorteilhafterweise mit entsprechenden Reagenzien verfügbar gemacht. Hierbei können sowohl Veränderungen des pHs (alkalisch), Hitze, Wiederholung extremer Temperaturveränderungen (Einfrieren/Auftauen), Veränderung der physiologischen Wachstumsbedingungen (Osmotischer Druck), Einwirkung von Detergenzien, chaotropen Salzen oder Enzymen (z. B. Proteasen, Lipasen), alleine oder in Kombination zur Freisetzung der Nukleinsäuren beitragen.

In einer ersten essentiellen Reaktion des erfindungsgemäßen Verfahrens werden kontaminierende Nukleinsäuren enzymatisch abgebaut. Verfahren, die dies bewerkstelligen, sind aus dem Stand der Technik bekannt, z. B. aus EP-A-0 401 037 und EP-A-0 415 755, auf die hier vollinhaltlich Bezug genommen wird. Hierzu werden in der Probe durch Zugabe von Reagenzien, die das Enzym enthalten, die Bedingungen eingestellt, die für den Abbau erforderlich sind. Die Zugabe kann prinzipiell einzeln, aber auch zusammen mit anderen Reagenzien geschehen. Als abbauendes Enzym können alle Enzyme eingesetzt werden, die bewirken, daß Nukleinsäuren, die nicht Gegenstand des aktuellen Nachweises oder der aktuellen Amplifikation sein sollen, nicht mehr als Templat zur Amplifikation zur Verfügung stehen, die zu amplifizierende Nukleinsäure aus der Probe jedoch nicht wesentlich beeinträchtigen. Dazu gehören beispielsweise Enzyme, die für die Amplifikationsprodukte charakteristische Eigenschaften oder Modifikationen erkennen, z. B. den Einbau spezieller Mononukleotide oder Oligonukleotide. Ein herausragendes Beispiel für ein solches Enzym ist die Uracil-N-Glycosilase, welche in die Amplifikate eingebautes Uracil erkennt und alle U-haltigen Nukleinsäuren soweit abbaut, daß sie nicht mehr als Template für die nachfolgende Amplifikation zur Verfügung stehen. Hierfür wird in der Amplifikationsreaktion anstelle des oder zusätzlich zu dem normalen dTTP oder dem entsprechenden Oligonukleotid dUTP oder ein U-haltiger Primer eingesetzt. Hierdurch werden die Amplifikate aus anderen Quellen als der aktuellen Probe chemisch differenzierbar von den zu amplifizierenden Nukleinsäuren der aktuellen Probe.

Die enzymatische Abbaureaktion wird solange geführt, bis voraussichtlich keine kreuzkontaminierenden Nukleinsäuren mehr in der Probe vorhanden sind. Danach wird das Enzym inaktiviert. Dies kann wiederum auf verschiedenste Weise geschehen, bevorzugt ist jedoch die thermische Inaktivierung, da dabei das Reaktionsgefäß nicht geöffnet werden muß. Hierzu wird die Reaktionsmischung auf eine Temperatur erhitzt, bei der das Enzym inaktiviert ist, das heißt, daß es vermutlich durch Änderung seiner räumlichen Anordnung so verändert ist, daß die enzymatische Aktivität verloren geht. Bei der Verwendung von hitzelabiler UNG ist eine Temperatur von 50°C, bevorzugt von 40°C bis 96°C hierfür ausreichend. Die hitzestabile UNG erfordert für die Inaktivierung eine höhere Temperatur. Bei den erfindungsgemäß bevorzugten, thermischen Amplifikationsverfahren kann die Inaktivierung im ersten Hitzeschritt des Amplifikationsverfahren geschehen, so daß ein separater Schritt entfallen kann. In diesem Fall ist es bevorzugt, die für die Amplifikation erforderlichen Reagenzien, d. h. insbesondere die (Mono)-Nukleosidtriphosphate, die Polymerase, die Primer und eventuell Puffer, schon zusammen, oder kurz vor oder nach der Zugabe des Abbauenzyms zur Probe zuzugeben. Besonders bevorzugt werden die für die Amplifikation erforderlichen, die für den Abbau erforderlichen und die für die Inaktivierung erforderlichen Reagenzien als eine Mischung oder Lösung zugegeben. Hierdurch erspart man sich Arbeitsschritte und erniedrigt die aktive und die passive Kontaminationsgefahr.

Unter einer Amplifikation im Sinne der Erfindung sind alle in-vitro-Verfahren zur Vermehrung von Nukleinsäuren oder Teilen davon zu verstehen, insbesondere auf der Durchführung von Thermozyklen beruhende Verfahren. Besonders bevorzugtes Amplifikationsverfahren ist die PCR. Auf die Offenbarung in US-A-4,683,202 wird hier voll Bezug genommen. Bevorzugt sind insbesondere solche Amplifikationsverfahren, bei denen unter Verwendung der zu amplifizierenden Nukleinsäure als Templatnukleinsäure unter Katalyse durch eine DNA-Polymerase, bevorzugt eine thermostabile Polymerase, besonders bevorzugt T. aq. (DNA) bzw. T.th.(RNA) Polymerase, Verlängerungsprodukte eines Primers gebildet werden, die wiederum als Templatnukleinsäure für die Verlängerung eines Primers zur Verfügung stehen. Durch zyklisch ablaufende Denaturierung der gebildeten Doppelstränge, Anhybridisierung von Primern und Verlängerung der Primer werden Teile der Nukleinsäure theoretisch exponentiell vermehrt.

Nukleosidtriphosphate (NTP) sind Ribo (rNTP)- oder Desoxyribonukleosidtriphosphate (dNTP). Sie können sowohl markiert als auch unmarkiert sein.

Eine Templatnukleinsäure ist eine Nukleinsäuren, zu der ein im wesentlichen komplementärer Nukleinsäurestrang neu gebildet wird. In Bezug auf die Sequenzinformation dient die Templatnukleisäure als Matrize für die Umschreibung.

Bevorzugt finden die Reaktionen in einem Kunststoffgefäß statt. Insbesondere sind dabei solche Gefäße einsetzbar, welche normalerweise in üblichen Thermocyclern als Amplifikationsgefäße verwendet werden. Beispiele sind Gefäße aus Polystyrol, Polyethylen oder Luran.

Das die Reaktivierung verhindernde Reagenz kann der Probe prinzipiell in jeder Stufe der Reaktion zugesetzt werden, jedoch vorteilhafterweise, bevor das Enzym Gelegenheit zur Reaktivierung hat, das heißt spätestens nach erfolgter Amplifikation, je nach Lagenmgstemperatur der Reaktionsmischung nach Amplifikation erfolgt die Reaktivierung unterschiedlich schnell, weshalb bei höheren Temperaturen ein früherer Zusatz sinnvoller ist als bei tieferen Temperaturen. Zuwarten erniedrigt die Ausbeute an Amplifikaten, da das Enzym die gerade amplifizierten Nukleinsäuren wieder abzubauen beginnt. Bevorzugt wird das Reagenz jedoch schon zusammen mit dem abbauenden Enzym zu der Probe zugegeben.

Als Reagenz haben sich insbesondere Substanzen mit einer hydrophoben Gruppe, besonders Detergenzien, als nützlich erwiesen. Es wurde nämlich gefunden, daß die Zurückfaltung des Enzyms durch solche Stoffe weitgehend unterdrückt werden kann. Dies gilt insbesondere für Detergenzien mit einem ausgeprägten hydrophoben Anteil von ca 5 bis 30 Kohlenstoffatomen, z. B. einem Fettsäurerest, wie bevorzugt einem Lauroylrest oder N-Acyl-Aminosäuren. Durch Anlagerung der hydrophoben Reste wird eine Restrukturierung vermutlich erschwert. Mögliche weitere Detergenzien sind beispielsweise LDS (Lithiumdodecylsulfat) oder SDS (Sodium-dodecylsulfat), wenngleich diese das Problem einer geringen Löslichkeit bei niedrigen Temperaturen haben. Besonders geeignet im Sinne der Erfindungen ist die Klasse der anionischen Detergenzien, insbesondere Lauroylsarkosin.

Wenn das Detergenz schon während der Amplifikation enthalten sein soll, muß auch darauf geachtet werden, daß das Detergenz die Aktivität der Polymerase nicht wesentlich beeinträchtigt. Dies kann der Fachmann aus der Gruppe der oben genannten Detergenzien durch einfache Versuche zur Amplifikation in Anwesenheit des gewählten Detergenz herausfinden. Eine leicht verminderte Amplinkationseffizienz kann auch durch Zugabe von etwas mehr Polymerase ausgeglichen werden.

Die Konzentration des Detergenz richtet sich auch nach dem Detergenz selbts, so daß für die gesamte Gruppe gültige Angaben nur sehr schwierig zu geben sind. Für N-Lauroylsarkosin (LS) z. B. hat es sich als zweckmäßig erwiesen, die Konzentration aus dem Bereich von zwischen 0.01 und 0.1 GEW-% (w/v), besonders bevorzugt 0,04 % und 0,05 %. zu wählen (Endkonzentration im PCR-Ansatz).

Das erfindungsgemäßen Verfahren hat den Vorteil, daß die resultierenden Reaktionsmischungen nach Amplifikation nicht sofort dem Nachweis zugeführt werden müssen, sondern auch einige Zeit bei Raumtemperatur gelagert werden können, ohne daß ein signifikanter Abbau der Amplifikationsprodukte stattfindet. Eine separate Zugabe von Reagenzien kann entfallen.

Das erfindungsgemäße Amplifiktionsverfahren kann verwendet werden, um eine ausreichende Menge an nachweisbaren Nukleinsäuren zu erzeugen. In Nachweisverfahren können die erfindungsgemäß gebildeten Amplifikate dann auf allgemein bekannte Art und Weise nachgewiesen werden. Dabei können auch schon während der Amplifikation nachweisbare Gruppen, z. B. Fluorezenzmarkierungen, in die Amplifikate eingebaut werden. Bevorzugt werden die Amplifikate mit einer markierten Sonde hybridisiert, an einer festen Phase abgefangen, gewünschtenfalls von einem eventuellen Überschuß an markierten Bestandteilen befreit und an der Festphase aufgrund der vorhandenen Markierung nachgewiesen. Eine Vielzahl von Formaten für solche Nachweise sind bekannt, z. B. ein Format, wie in EP-A-0 324 474 beschrieben.

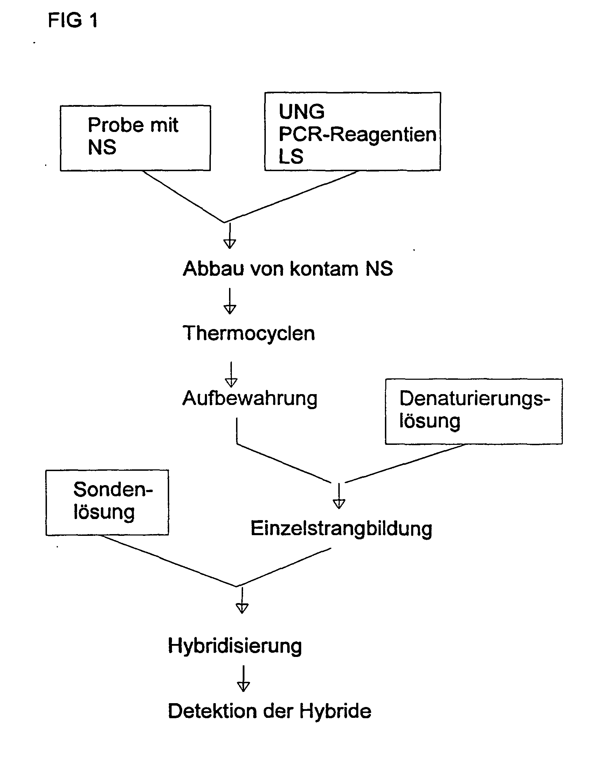

In einer besonders bevorzugten Ausführungsform (siehe hierzu FIG 1) wird der Probe, z.B. einem Serum nach Probenvorbereitung, ein Reagenz enthaltend UNG, LS, Mononukleosidtriphosphatgemisch (enthaltend auch dUTP), T.th.-Polymerase und HIV-spezifischen Biotinmarkierten Primern zugegeben. Das Gefäß wird verschlossen und eine Weile inkubiert. Dann wird das Gefäß in einen Thermocycler gebracht und erhitzt, solange wie es die Thermocyclen erfordern. Anschließend wird das Gefäß geöffnet, die Nukleinsäure denaturiert und an schließend eine rutheniummarkierte Detektorsonde sowie eine Suspension von streptavidinbeschichteten Magnetbeads zugegeben. Nach Mischen wird das Gemisch in eine Meßzelle für eine Elektrochemilumineszemmessung gebracht. Das gemessene Signal ist ein Zeichen für die Menge oder die Anwesenheit von HIV-Nukleinsäuren in der Probe.

Ebenfalls Gegenstand der Erfindung ist ein Reagenzkit zur Amplifikation von Nukleinsäuren, enthaltend in einem oder mehreren Behältern Reagenzien zur Amplifikation von Nukleinsäuren, ein Nukleinsäuren abbauendes Enzym sowie ein die Reaktivierung des Enzyms verhinderndes Detergenz.

Ebenfalls Gegenstand der Erfindung ist ein Reagenz zur Amplifikation von Nukleinsäuren enthaltend in einer Lösung
- mindestens ein Reagenz ausgewählt aus der Gruppe Nukleosidtriphosphate, Primer, Polymerase,
- ein Nukleinsäure abbauendes Enzym, und
- ein die Reaktivierung des Nukleinsäure abbauenden Enzyms verhinderndes Detergenz.

Ebenfalls Gegenstand der Erfindung ist die Verwendung von Lauroylsarcosin zur Verhinderung der Reaktivierung von Uracil-N-Clycosilase.

Im Rahmen der Erfindung wurde ebenso gefunden, daß es nicht erforderlich ist, daß das die Reaktivierung des Enzyms verhindernde Detergenz schon vor Amplifikation der Probe zuzusetzen. Wirklich erforderlich ist die Zugabe erst dann, wenn das Enzym Gelegenheit zu einer Reaktivierung hat, d. h. z. B. einer Abkühlung unter die Temperatur, bei der eine Rückfaltung stattfindet. Dies bedeutet, daß das Detergenz auch nach Durchführung der Amplifikation zugesetzt werden kann. Für diesen Fall ist, ebenso wie für die vorher geschilderte Ausführungsform, bevorzugt ein Detergenz zu benutzen, welches die Enzymäktivität nicht von sich aus deaktiviert, aber die Reaktivierung aus einem nicht aktiven Zustand verhindert.

Im Rahmen dieser Untersuchungen wurde ferner festgestellt, daß Detergenzien in Verfahren zum Nachweis von Nukleinsäuren mit den Schritten in-vitro-Herstellung einer Vielzahl von Kopien eines Teilbereiches der Nukleinsäure und Nachweis der Kopien als Maß für die Anwesenheit oder die Menge der nachzuweisenden Nukleinsäure dadurch verbessert werden können, daß der Lösung mit den Kopien vor dem Nachweis ein Detergenz zugesetzt wird. Das Detergenz führt wohl zu einer Reduktion verwünschter Nebenreaktionen von DNA-Polymerasen bei niedrigen Temperaturen, z. B. Auffüllreaktionen an einzelsträngigen Templaten. Diese Nebenreaktionen können die Performance des Nachweisverfahrens erheblich beeinträchtigen.

Ein besonderer Gegenstand ist ein Verfahren zur Amplifikation von Nukleinsäuren in einer Probe enthaltend die Schritte Behandlung der Probe mit einem Enzym unter Bedingungen, bei denen Produkte aus Amplifikationen von Nukleinsäuren anderer Proben enzymatisch abgebaut werden, Inaktivierung des Enzyms und Behandlung der Probe unter Bedingungen, bei denen die Nukleinsäuren amplifiziert werden, wobei der Probe nach Amplifikation eine Detergenz zugesetzt wird, welches die Enzymaktivität des aktiven Enzyms nicht beeinträchtigt aber die Reaktivierung des Enzyms verhindert.

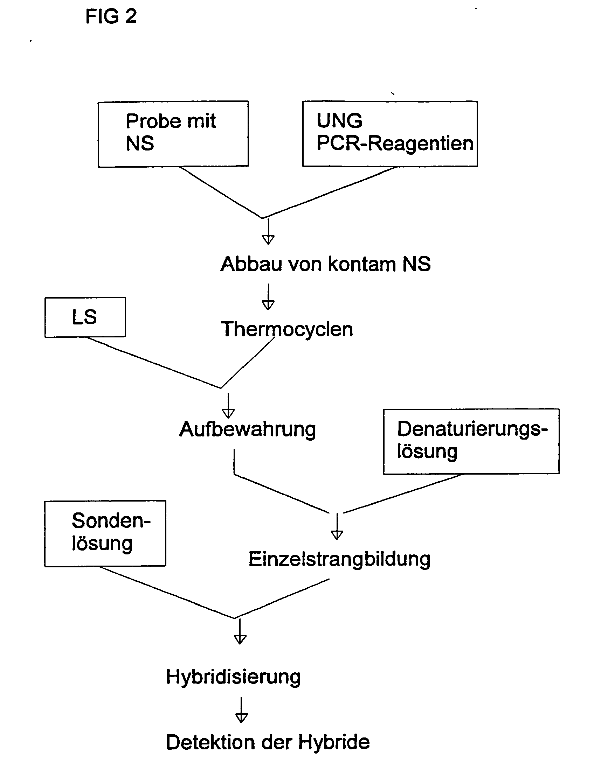

Ein solches Verfahren ist schematisch in FIG 2 gezeigt. In dieser Ausführungsform wird zunächst die Probe, die die Nukleinsäure (NS) enthält, mit der UNG und den PCR-Reagenzien in Kontakt gebracht. Dabei geschieht der Abbau von kontaminierenden Nukleinsäuren. Anschließend wird die Reaktionsmischung den Thermozyklen unterzogen. Nach Amplifikation wird das Reagenz (hier Lauroylsarcosin, LS) zugesetzt. Die resultierende Mischung kann nun so lange aufbewahrt werden, bis eine Denaturierungslösung zur Einzelsträngigmachung der Amplifikate zugegeben werden soll. Danach wird eine Lösung mit einer Detektorsonde zugegeben, Hybridisierungsbedingungen eingestellt und gegebenenfalls die Hybride detektiert.

Die folgenden Beispiele sollen die Erfindung näher erläutern:

### Beispiel 1:

### Einfluß von LS auf die Amplifikationseffizienz und die UNG-Reaktivierung bei Zugabe zum Mastermix am Beispiel Chlamydia (DNA-PCR mit Taq-Polymerase):

| Reagenzien | Endkonzentration im Mastermix |
|---|---|
| 10x PCR-Puffer | 1x |
| MgCl₂ | 2.5 mM |
| Taq-Polym. | 5u |
| dNTP-Mix | 200µM (dATP, dCTP, dGTP) / 600µM (dUTP) |
| UNG | 2u |
| LS | 0.05% (optional) |
| Primer forward | 0.3µM (5'-cctcttccccagaacaataagaacac-3', SEQ.ID.NO.1) |
| Primer reverse | 0.3µM (5'-bio-gggattcctgtaacaacaagtcagg-3', SEQ.ID.NO.2) |

LS: N-Lauroyl-Sarkosin (Applichem, BRD, Best.-Nr. A1163)

Die Amplifikation wurde nach folgendem Cyclerprotokoll ausgeführt:

| | | |
|---|---|---|
| 10min | 37°C | Dekontamination durch UNG |
| 5min | 95°C | Denaturierung |
| 1min | 60°C | Primer-Annealing und Elongation |
| 30sec | 95°C | 29 Zyklen Amplifikation |
| 1 min | 60°C | |

Amplifiziert wurden in Mehrfachbestimmungen 10¹ und 10² Kopien Chlamydia-Plasmid. Die Amplifikation erfolgte jeweils mit und ohne LS (Kontrolle) im Mastermix. Nach Amplifikation wurden alle Proben gemessen (t₀), anschließend für 3h bei 30°C inkubiert (t₃ₕ) und dann erneut gemessen (ECL-Detektion, Elecsys®1010, Boehringer Mannheim GmbH). Zur Hybridisierung wurde eine Ruthenium-gelabelte DNA-Sonde verwendet (5'-Ru-catagcactatagaactctg-3', SEQ.ID.NO. 3).

### Ergebnis:

| **Chlamydia-Plasmid** | **ohne LS** | | **mit LS** | |
|---|---|---|---|---|
| | t₀ | t₃ₕ | t₀ | t₃ₕ |
| 10² Kopien | 377 | 58 | 401 | 385 |
| 10² Kopien | 342 | 61 | 286 | 253 |
| 10² Kopien | 409 | 49 | 278 | 270 |
| 10² Kopien | 441 | 42 | 289 | 301 |
| 10² Kopien | 279 | 53 | 421 | 399 |
| 10² Kopien | 313 | 37 | 345 | 328 |
| 10² Kopien | 446 | 68 | 499 | 479 |
| 10² Kopien | 364 | 59 | 331 | 296 |
| 10¹ Kopien | 13 | 9 | 51 | 29 |
| 10¹ Kopien | 43 | 9 | 16 | 12 |
| 10¹ Kopien | 15 | 8 | 18 | 15 |
| 10¹ Kopien | 19 | 8 | 99 | 61 |
| 10¹ Kopien | 23 | 9 | 207 | 136 |
| 10¹ Kopien | 25 | 8 | 51 | 73 |
| 10¹ Kopien | 43 | 8 | 37 | 22 |
| 10¹ Kopien | 48 | 9 | 34 | 19 |
| 10¹ Kopien | 22 | 7 | 30 | 19 |
| 10¹ Kopien | 37 | 9 | 56 | 35 |

- Die Ergebnisse zeigen, daß LS kaum Einfluß auf die Amplifikationseffizienz der Taq-Polymerase hat. Die Signale liegen bei gleichen Kopienzahlen auch in der gleichen Größenordnung nach Detektion, unabhängig davon, ob mit oder ohne LS amplifiziert wurde.
- Andererseits besitzt LS, insbesondere bei niedrigen Kopienzahlen, einen sehr positiven Effekt auf die Amplifikatstabilität. Definiert man einen Wert (cutoff), unterhalb dessen individuelle Proben als negativ gefunden werden, so erkennt man am Beispiel der niedrigen Kopienzahl von 10 Kopien, daß zunächst alle Proben als positiv klassifiziert werden, nach 3h Inkubation (30°C) jedoch sämtlich negativ gefunden würden. Durch Zugabe von LS im Reaktionsmix wird dieser Effekt vollständig vermieden. Hierdurch ist die Ergebnissicherheit deutlich erhöht!

### Beispiel 2:

### Einfluß von LS auf die Dekontaminationsleistung:

Um die Dekontaminationsleistung zu testen, wurde zunächst eine artifizielle Kontaminationsreihe von 10¹ bis 10⁶ Kopien Uracil-haltiges Amplifikat (Substrat für die UNG) hergestellt. Diese wurde anschließend nach obigem Mastermix und PCR-Protokoll amplifiziert und die Ergebnisse hinsichtlich Dekontamination/Reamplifikation untersucht.

| **Chlamydia-dUTP-** | **ohne UNG** | **mit UNG** | **mit UNG** |
|---|---|---|---|
| **Amplifikat** | | **mit LS** | **ohne LS** |
| 10¹ Kopien | 4 | 4 | 4 |
| 10² Kopien | 9 | 4 | 4 |
| 10³ Kopien | 402 | 9 | 6 |
| 10⁴ Kopien | 912 | 30 | 62 |
| 10⁵ Kopien | 3091 | 145 | 221 |
| 10⁶ Kopien | 4243 | 638 | 783 |

- LS hat keinen Einfluß auf die Dekontaminationsleistung der UNG zu Beginn der Amplifikation. In beiden Fällen (+/- LS) ergibt sich eine Dekontamination von mindestens 10³ Kopien Amplifikat.

### Beispiel 3

### Einfluß von LS auf die Signalstabilität bei Zugabeweise nach PCR am Beispiel HIV (RNA-PCR mit Tth-Polymerase):

| Reagenzien | Endkonzentration im Mastermix |
|---|---|
| 5xRT-PCR-PuSer | 1x |
| MnOAc | 1,25 mM |
| Tth-Polym. | 15u |
| dNTP-Mix | 200µM (dATP, dCTP, dGTP) / 600µM (dUTP) |
| UNG | 2u |
| Primer forw. SK462 | 0.2µM (AGTTGGAGGACATCAAGCAGCCATGCAAAT; |
| | SEQ.ID.NO. 4) |
| Primer rev. SK431-bio | 0.2µM (TGCTATGTCAGTTCCCCTTGGTTCTCT, |
| | SEQ.ID.NO. 5) |

Die Amplifikation wurde nach folgendem Cyclerprotokoll ausgeführt:

| | | | |
|---|---|---|---|
| | 10min | 37°C | Dekontamination durch UNG |
| | 30min | 60°C | reverse Transkription |
| 4 Zyklen: | 10sec | 95°C | Denaturierung |
| | 10sec | 55°C | Primer-Annealing |
| | 10sec | 72°C | Elongation |
| 26 Zyklen: | 10sec | 90°C | Denaturierung |
| | 10sec | 60°C | Primer-Annealing |
| | 10sec | 72°C | Elongation |

Amplifiziert wurden in Mehrfachbestimmungen 10², 10⁴ und 10⁵ Kopien HIV-RNA-Standard. Nach Amplifikation wurden zunächst alle Proben geteilt und gemessen (t₀), anschließend die Hälfte der Proben mit 0,05% LS (w/v, Endkonzentration) abgestoppt und alle Proben für 3h bei 30°C inkubiert (t₃ₕ) und dann erneut gemessen (ECL-Detektion, Flash-Symu). Zur Hybridisierung wurde eine Ruthenium-gelabelte DNA-Sonde verwendet. (SK102b, ATCAATGAGGAAGCTGCAGA, SEQ.ID.NO. 6).

### Ergebnis:

| **HIV-wt-RNA** | **ohne LS** | | **mit LS** | |
|---|---|---|---|---|
| | t₀ | t₃ₕ | to | t₃ₕ |
| 10⁵ Kopien | 3204 | 2898 | 3167 | 3162 |
| 10⁵ Kopien | 3095 | 2924 | 2987 | 3033 |
| 10⁵ Kopien | 3003 | 2992 | 3053 | 3053 |
| 10⁵ Kopien | 3118 | 2944 | 3091 | 3056 |
| 10⁴ Kopien | 1307 | 1105 | 1311 | 1300 |
| 10⁴ Kopien | 1310 | 1177 | 1287 | 1249 |
| 10⁴ Kopien | 1295 | 1120 | 1284 | 1328 |
| 10⁴ Kopien | 1317 | 1111 | 1350 | 1284 |
| 10² Kopien | 205 | 189 | 204 | 200 |
| 10² Kopien | 207 | 195 | 201 | 203 |
| 10² Kopien | 208 | 194 | 205 | 202 |
| 10² Kopien | 200 | 188 | 199 | 200 |

- Die Ergebnisse zeigen, daß die Inkubation der Amplifikate ohne LS zu einer schwachen aber signifikanten Signalabnahme führt. Diese wird durch die Zugabe von N-Lauroylsarcosin komplett vermieden.

## Patentansprüche

1. Verfahren zur Amplifikation von Nukleinsäuren in einer Probe enthaltend die Schritte
- Behandlung der Probe mit einem Enzym unter Bedingungen, bei denen Produkte aus Amplifikationen von kreuzkontaminierenden Nukleinsäuren anderer Proben enzymatisch abgebaut werden,
- Inaktivierung des Enzyms, und
- Behandlung der Probe unter Bedingungen, bei denen die Nukleinsäuren amplifiziert werden,
**dadurch gekennzeichnet, daß** die Amplifikation in Anwesenheit eines die Reaktivierung des Enzyms verhindernden Detergenz mit einer hydrophoben Gruppe stattfindet.

2. Verfahren gemäß Anspruch 1, **dadurch gekennzeichnet, daß** das Enzym Uracil-N-Glycosilase ist.

3. Verfahren gemäß einem der vorangehenden Ansprüche, **dadurch gekennzeichnet, daß** das Detergenz mit einer hydrophoben Gruppe ein anionisches Detergenz ist.

4. Verfahren gemäß einem der vorangehenden Ansprüche, **dadurch gekennzeichnet, daß** das Detergenz mit einer hydrophoben Gruppe Lauroylsarcosin ist.

5. Verfahren gemäß einem der vorangehenden Ansprüche, **dadurch gekennzeichnet, daß** das Detergenz mit einer hydrophoben Gruppe der Probe zusammen mit den Reagenzien für die Amplifikation der Nukleinsäuren zugesetzt wird.

6. Verfahren gemäß Anspruch 5, **dadurch gekennzeichnet, daß** auch das Enzym der Probe zusammen mit den Reagenzien für die Amplifikation zugesetzt wird.

7. Verfahren gemäß einem der vorangehenden Ansprüche, **dadurch gekennzeichnet, daß** die Konzentration des Detergenz mit einer hydrophoben Gruppe so gewählt wird, daß besagtes Detergenz die Abbau-Aktivität des Enzyms in nativem Zustand nicht wesentlich beeinflußt, es aber die Reaktivierung nach einer Deaktivierung im wesentlichen verhindert.

8. Verfahren gemäß Anspruch 7, **dadurch gekennzeichnet, dass** das Detergenz mit einer hydrophoben Gruppe die Amplifikation nicht wesentlich stört.

9. Reagenzkit zur Amplifikation von Nukleinsäuren, enthaltend Reagenzien zur Amplifikation von Nukleinsäuren, Uracil-N-Glycosilase sowie ein Detergenz mit einer hydrophoben Gruppe.

10. Verwendung von N-Lauroylsarcosin zur Verhinderung der Reaktivierung von Uracil-N-Glycosilase.

11. Reagenz zur Amplifikation von Nukleinsäuren enthaltend in einer Lösung
- mindestens ein Reagenz ausgewählt aus der Gruppe Nukleosidtriphosphate, Primer, Polymerase,
- Uracil-N-Glycosilase, und
- ein Detergenz mit einer hydrophoben Gruppe.

12. Verfahren zum Nachweis einer Nukleinsäure enthaltend
- Amplifikation der Nukleinsäure gemäß einem der Ansprüche 1 bis 8 und
- Nachweis der gebildeten Amplifikate als Zeichen für die Anwesenheit oder die Menge an Nukleinsäure in der Probe.

13. Verfahren zur Amplifikation von Nukleinsäuren in einer Probe enthaltend die Schritte
- Behandlung der Probe mit einem Enzym unter Bedingungen, bei denen Produkte aus Amplifikationen von Nukleinsäuren aus anderen Proben enzymatisch abgebaut werden,
- Inaktivierung des Enzyms, und
- Behandlung der Probe unter Bedingungen, bei denen die Nukleinsäuren amplifiziert werden,
**dadurch gekennzeichnet, daß** der Probe nach der Amplifikation ein Detergenz mit einer hydrophoben Gruppe zugesetzt wird, welches die Enzymaktivität des aktiven Enzyms nicht beeinträchtigt, aber die Reaktivierung des Enzyms verhindert.

## Claims

1. Method for amplifying nucleic acids in a sample comprising the steps of
- treating the sample with an enzyme under conditions in which products from amplifications of cross-contaminating nucleic acids from other samples are enzymatically degraded,
- inactivating the enzyme, and
- treating the sample under conditions in which the nucleic acids are amplified,
**characterized in that** the amplification takes place in the presence of a detergent with a hydrophobic group which prevents the reactivation of the enzyme.

2. Method according to claim 1, **characterized in that** the enzyme is uracil-N-glycosylase.

3. Method according to one of the previous claims, **characterized in that** the detergent with a hydrophobic group is an anionic detergent.

4. Method according to one of the previous claims, **characterized in that** the detergent with a hydrophobic group is lauroylsarcosine.

5. Method according to one of the previous claims, **characterized in that** the detergent with a hydrophobic group is added to the sample together with the reagents for the amplification of the nucleic acids.

6. Method according to claim 5, **characterized in that** the enzyme is also added to the sample together with the reagents for the amplification.

7. Method according to one of the previous claims, **characterized in that** the concentration of the detergent with a hydrophobic group is selected such that the said detergent does not substantially influence the degradation activity of the enzyme in the native state but it substantially prevents its reactivation after a deactivation.

8. Method according to claim 7, **characterized in that** the detergent with a hydrophobic group does not substantially interfere with the amplification.

9. Reagent kit for amplifying nucleic acids containing reagents for amplifying nucleic acids, uracil-N-glycosylase as well as a detergent with a hydrophobic group.

10. Use of N-lauroylsarcosine to prevent reactivation of uracil-N-glycosylase.

11. Reagent for amplifying nucleic acids containing in a solution
- at least one reagent selected from the group nucleoside triphosphates, primers, polymerase,
- uracil-N-glycosylase, and
- a detergent with a hydrophobic group.

12. Method for detecting a nucleic acid comprising
- amplifying the nucleic acid according to one of the claims 1 to 8 and
- detecting the amplificates that are formed as an indication for the presence or amount of nucleic acid in the sample.

13. Method for amplifying nucleic acids in a sample comprising the steps of
- treating the sample with an enzyme under conditions in which products from amplifications of nucleic acids from other samples are enzymatically degraded,
- inactivating the enzyme, and
- treating the sample under conditions in which the nucleic acids are amplified
**characterized in that** a detergent with a hydrophobic group which does not impair the enzyme activity of the active enzyme but prevents the reactivation of the enzyme is added to the sample after the amplification.

## Revendications

1. Procédé pour l'amplification d'acides nucléiques dans un échantillon, comprenant les étapes
- de traitement de l'échantillon avec une enzyme dans des conditions dans lesquelles des produits issus d'amplifications d'acides nucléiques d'autres échantillons, générant une contamination croisée, sont soumis à une dégradation par voie enzymatique ;
- d'inactivation de l'enzyme ; et
- de traitement de l'échantillon dans des conditions dans lesquelles les acides nucléiques sont amplifiés ;
**caractérisé en ce que** l'amplification a lieu en présence d'un détergent comprenant un groupe hydrophobe, empêchant la réactivation de l'enzyme.

2. Procédé selon la revendication 1, **caractérisé en ce que** l'enzyme est l'uracil-N-glycosylase.

3. Procédé selon l'une quelconque des revendications précédentes,
**caractérisé en ce que** le détergent comprenant un groupe hydrophobe est un détergent anionique.

4. Procédé selon l'une quelconque des revendications précédentes,
**caractérisé en ce que** le détergent comprenant un groupe hydrophobe est la lauroylsarcosine.

5. Procédé selon l'une quelconque des revendications précédentes,
**caractérisé en ce que** le détergent comprenant un groupe hydrophobe est ajouté à l'échantillon de manière conjointe avec les réactifs pour l'amplification des acides nucléiques.

6. Procédé selon la revendication 5, **caractérisé en ce que** l'enzyme est également ajoutée à l'échantillon de manière conjointe avec les réactifs pour l'amplification.

7. Procédé selon l'une quelconque des revendications précédentes,
**caractérisé en ce que** la concentration du détergent comprenant un groupe hydrophobe est sélectionnée de telle sorte que ledit détergent n'influence pas de manière essentielle l'activité de dégradation de l'enzyme à l'état naturel, mais empêche essentiellement la réactivation après une désactivation.

8. Procédé selon la revendication 7, **caractérisé en ce que** le détergent comprenant un groupe hydrophobe ne perturbe pas l'amplification de manière essentielle.

9. Nécessaire de réactifs pour l'amplification d'acides nucléiques, contenant des réactifs pour l'amplification d'acides nucléiques, l'uracil-N-glycosylase ainsi qu'un détergent comprenant un groupe hydrophobe.

10. Utilisation de la N-lauroylsarcosine pour empêcher la réactivation de l'uracil-N-glycosylase.

11. Réactif pour l'amplification d'acides nucléiques, contenant, en solution
- au moins un réactif choisi parmi le groupe contenant des nucléosides triphosphates, des amorces, des polymérases ;
- l'uracil-N-glycosylase ; et
- un détergent comprenant un groupe hydrophobe.

12. Procédé pour le décèlement d'un acide nucléique, comprenant
- l'amplification de l'acide nucléique selon l'une quelconque des revendications 1 à 8 ; et
- le décèlement des produits d'amplification obtenus comme indication pour la présence ou la quantité de l'acide nucléique dans l'échantillon.

13. Procédé pour l'amplification d'acides nucléiques dans un échantillon, comprenant les étapes
- de traitement de l'échantillon avec une enzyme dans des conditions dans lesquelles des produits issus d'amplifications d'acides nucléiques d'autres échantillons sont soumis à une dégradation par voie enzymatique ;
- d'inactivation de l'enzyme ; et
- de traitement de l'échantillon dans des conditions dans lesquelles les acides nucléiques sont amplifiés ;
**caractérisé en ce qu'**après l'amplification, on ajoute à l'échantillon un détergent comprenant un groupe hydrophobe qui n'entrave pas l'activité enzymatique de l'enzyme active, mais qui empêche la réactivation de l'enzyme.
